# EUROPÄISCHE PATENTSCHRIFT

(11) **EP 1 853 248 B1**
(45) Veröffentlichungstag und Bekanntmachung des Hinweises auf die Patenterteilung: **29.10.2008**
(21) Anmeldenummer: 06707381.7
(22) Anmeldetag: 02.03.2006
(51) Int. Cl.: A61K 31/205, A23K 1/16, A23L 1/30

(54) **Verwendung der SALZE, ANLAGERUNGS- UND KOMPLEXVERBINDUNGEN DER GUANIDINOESSIGSÄURE**
Use of the SALTS, ADDITION COMPOUNDS AND COMPLEX COMPOUNDS OF GUANIDINOACETIC ACID
Utilisation des SELS ET COMPOSES D'ADDITION ET COMPLEXES DE L'ACIDE GUANIDINOACETIQUE

(30) Priorität: 04.03.2005 DE 102005009990
(43) Veröffentlichungstag der Anmeldung: 14.11.2007
(73) Patentinhaber: AlzChem Trostberg GmbH, 83308 Trostberg (DE)
(72) Erfinder: GASTNER, Thomas, 84549 Engelsberg (DE); KRIMMER, Hans-Peter, 84558 Kirchweidach (DE)
(74) Vertreter: Dey, Michael
(86) Internationale Anmeldenummer: PCT/EP2006/001908
(87) Internationale Veröffentlichungsnummer: WO 2006/092298

(56) Entgegenhaltungen:
- WO-A-01/00203
- WO-A-91/07954
- WO-A-20/04000297
- WO-A-20/05120246
- GB-A- 1 195 199
- GB-A- 1 195 200
- US-A- 5 939 078
- DE MIRANDA L ET AL: "Study on guanidino-carboxylate interactions with copper(II) ternary complexes of guanidinoacetic acid with glutamic and aspartic acid" POLYHEDRON, Bd. 22, Nr. 2, 2003, Seiten 225-233, XP002377267

## Beschreibung

Gegenstand der vorliegenden Erfindung ist die Bereitstellung von neuen Salzen, Anlagerungs- und Komplexverbindungen der Guanidinoessigsäure. Diese besitzen verbesserte physiologische und therapeutische Eigenschaften und eignen sich für die Verwendung als Nahrungsergänzungsmittel, als Futtermittel und in kosmetischen oder dermatologischen Zubereitungen, wobei insbesondere die ausgeprägte Stabilität und gute Bioverfügbarkeit der Salze im Vordergrund stehen.

Guanidinoessigsäure ist eine im Menschen vorkommende körpereigene Substanz, welche bei der Biosynthese des Kreatins eine zentrale Rolle einnimmt. Kreatin ist für den Energiestoffwechsel der Zelle von großer Bedeutung, wobei es sowohl durch die Nahrung aufgenommen, als auch vom Körper selbst gebildet werden kann. Die Biosynthese geht von Glycin und L-Arginin aus. Bei Säugetieren wird vor allem in den Nieren, aber auch in der Leber und der Bauchspeicheldrüse, durch das Enzym Aminotransferase die Guanidino-Gruppe des L-Arginins gespalten und eine N-C-N-Gruppe auf das Glycin übertragen. Das L-Arginin wird hierbei in L-Ornithin umgewandelt. Die so gebildete Guanidinoessigsäure wird im nächsten Schritt, bei Vertebraten geschieht dies ausschließlich in der Leber, mit Hilfe des Enzyms Transmethylase in Kreatin umgewandelt.

Kreatin ist als energiereiches Phosphokreatin neben dem Adenosintriphosphat (ATP) eine wesentliche Energiereserve des Muskels. Im Ruhezustand des Muskels kann ATP auf Kreatin eine Phosphat-Gruppe übertragen, wobei Phosphokreatin gebildet wird, welches dann im direkten Gleichgewicht mit ATP steht. Bei Muskelarbeit ist es von entscheidender Bedeutung, die ATP-Vorräte schnellstmöglich wieder aufzufüllen. Hierfür steht in den ersten Sekunden maximaler Muskelbelastung das Phosphokreatin zur Verfügung. Dieses kann in einer sehr schnellen Reaktion, durch das Enzym Kreatinkinase, eine Phosphatgruppe auf Adenosindiphosphat übertragen und somit ATP zurückbilden. Dies wird auch als Lohmann-Reaktion bezeichnet.

In den neunziger Jahren wurde Kreatin-Monohydrat aufgrund seiner einzigartigen Funktion im Energiestoffwechsel zu einem populären Nahrungsergänzungsmittel. Die Sport-Industrie nutzt Kreatin-Monohydrat, um den Pool an energiereichen Phosphatverbindungen im Körper während des Trainings zu erhöhen und den Body-Mass-Index zu verbessern. Neuere Studien mit Kreatin haben für diverse klinische Anwendungen positive therapeutische Resultate ergeben [Persky, A.M.; Brazeau, G.A.: Clinical Pharmacology of the Dietary Supplement Creatine Monohydrate. In: Pharmacol Rev, 2001, 53, 161-176]. Neben dem Kreatin selbst, also dem Kreatin-Monohydrat, haben sich zwischenzeitlich aber auch zahlreiche Kreatinsalze, wie das Kreatin-ascorbat, -citrat, -pyruvat und andere, ebenfalls als geeignete Nahrungsergänzungsmittel oder therapeutische Mittel erwiesen. Stellvertretend seien an dieser Stelle das europäische Patent EP 894 083 und die deutsche Offenlegungsschrift DE 197 07 694 A1 genannt.

In einer Reihe wissenschaftlicher Arbeiten konnte gezeigt werden, dass Kreatin und seine Salze zu einer Steigerung der fettfreien Muskelmasse und der Muskelleistung führt. So ist auch bekannt, dass die Bauchspeicheldrüse unter dem Einfluss von Guanidinverbindungen wie Kreatin und Guanidinoessigsäure vermehrt Insulin ausschüttet, wobei Guanidinoessigsäure die Ausschüttung von Insulin noch wesentlich besser als das Kreatin selbst stimuliert. Insulin fördert die Aufnahme von Glucose und Aminosäuren in die Muskelzelle und fördert somit die Proteinsynthese. Vorteilhaft ist auch, dass das Insulin die Aufnahme von Kreatin in den Muskel katalysiert. Weiterhin vermindert Insulin die Abbaurate der Muskulatur.

Positive Effekte wurden auch bei Tieren gefunden und Kreatin-Monohydrat wurde deshalb für die Verwendung als Futtermittelzusatz und als Fleischmehlersatz in der Tierernährung empfohlen. Seit dem Verbot von tierischen Eiweißen in Futtermitteln ab dem Jahre 2000 in der EU wurden viele Diäten für Zucht- und Masttiere auf rein vegetarische Diäten umgestellt, wobei auch weitgehend auf Fischmehl verzichtet wurde, welches in dem Verbot nicht eingeschlossen ist. Die Umstellung auf rein vegetarische Diäten führte zu Einbußen in der Leistung und auch nach nahezu fünf Jahren sind die rein vegetarischen Diäten denen mit tierischen Eiweißen unterlegen. Ein Grund dieser Unterlegenheit ist der Mangel an Kreatin. Frühere Versuche konnten klar zeigen, dass dem Futter zugesetztes Kreatin-Monohydrat die Leistung verbessern kann, wenn rein vegetarische Diäten verfüttert werden [Wallimann, T.; Pfirter, H.P.: Use of Creatine as a Feed Additive. EP1051914].

Neben den unzweifelhaft positiven Effekten besitzt Kreatin-Monohydrat auch einige Nachteile. Die Stabilität dieser Verbindung in wässerigen Lösungen ist sehr begrenzt und Kreatin-Monohydrat besitzt nach oraler Aufnahme nur eine geringe Bioverfügbarkeit. Weiterhin ist Kreatin-Monohydrat eine sehr teure Substanz und die erzielten Leistungsverbesserungen im Tiermastbereich werden nahezu durch die Kosten kompensiert.

In jüngster Zeit wurde deshalb auch Guanidinoessigsäure als Nahrungsergänzungsmittel und Futtermittel eingesetzt, welche im Vergleich zum Kreatin in wässriger Lösung eine erstaunliche Stabilität besitzt und wesentlich besser bioverfügbar ist. Die Guanidinoessigsäure wird im Körper sehr effizient und schnell in Kreatin umgewandelt. Guanidinoessigsäure kann deshalb bei gleicher Wirkung in wesentlich geringeren Mengen als Kreatin verabreicht werden. In einer Studie wurden an Ratten Diäten mit ca. 0,36 g/kg Guanidinoessigsäure verfüttert, wodurch sich der Kreatin-Gehalt im Muskel um 39 % gegenüber der Vergleichsgruppe erhöhte [Stead, L.M.; Au, K.P.; Jacobs, R.L.; Brosnan, M.E.; Brosnan, J.T.: Methylation demand and homocysteine metabolism: Effects of Dietary Provision of Creatine and Guanidinoacetate. In: Am. J. Physiol. Endocrinol. Metab, 2001 Nov; 281(5); 1095-100]. Die Zunahme des Kreatins im Muskel ist mit einer hohen Umwandlungsrate der aufgenommenen Guanidinoessigsäure in Kreatin zu erklären. Dies deckt sich auch mit der Beobachtung, dass das Enzym Transmethylase in sehr hohen Konzentrationen in der Leber gefunden wird.

Neben ihrem Einsatz als Nahrungsergänzungsmittel bzw. als Futtermittelzusatz ist Guanidinoessigsäure auch für kosmetische Anwendungszwecke geeignet. So beschreibt die WO 2001/000 203 A1 Guanidinoessigsäure als energielieferndes System und Antioxidans für die oberen Hautschichten, wobei die Guanidinoessigsäure hauptsächlich in Form von Cremes aufgebracht wird, wodurch die Haut vor ungünstigen Einflüssen wie Sonnenstrahlung und Stress geschützt wird.

Neben den Vorteilen der Guanidinoessigsäure gegenüber dem Kreatin besitzt die Verbindung hingegen den Nachteil einer sehr schlechten Löslichkeit in Wasser (1 g in 278 ml Wasser bei 15 °C).

Aus den bzgl. Guanidinoessigsäure geschilderten Nachteilen des Standes der Technik hat sich für die vorliegende Erfindung die Aufgabe gestellt, die Löslichkeit von Guanidinoessigsäure in Wasser zu verbessern und die Bioverfügbarkeit weiter zu erhöhen, wobei die bekannt guten physiologischen Eigenschaften der Guanidinoessigsäure erhalten werden sollten.

Gelöst wird diese Aufgabe durch die Bereitstellung von neuen, stabilen Salzen und/oder Anlagerungs- und/oder Komplexverbindungen der Guanidinoessigsäure mit Äpfelsäure, Asparaginsäure, Ascorbinsäure, Bernsteinsäure, Brenztraubensäure, Fumarsäure, Gluconsäure, α-Ketoglutarsäure, Oxalsäure, Pyroglutaminsäure, 3-Nicotinsäure, Milchsäure, Zitronensäure, Maleinsäure, Schwefelsäure, Essigsäure, Ameisensäure, 2-Hydroxybenzoesäure, L-Carnitin, Acetyl-L-Carnitin, Taurin, Betain, Cholin, Methionin und Liponsäure sowie als Natrium-, Kalium- oder Calciumguanidinoacetat.

Überraschend hat sich herausgestellt, dass nicht nur die Aufgabenstellung erfüllt werden konnte, da die beanspruchten Salze und/oder Anlagerungs- und/oder Komplexverbindungen gegenüber der Guanidinoessigsäureeine deutlich höhere Wasserlöslichkeit besitzen, sondern dass die neuen Verbindungen hinsichtlich ihrer Stabilität und Bioverfügbarkeit der Guanidinoessigsäuremindestens ebenbürtig sind.

Neben den neuen Verbindungen der Guanidinoessigsäure betrifft die vorliegende Erfindung auch eine Zusammensetzung mit physiologischer Wirksamkeit, die mindestens eines der beschriebenen Salze und/oder Anlagerungs- und/oder Komplexverbindungen der Guanidinoessigsäure gemäß vorliegender Erfindung als wirksamen Inhaltsstoff enthält.

Von der vorliegenden Erfindung ist auch die Verwendung dieser Zusammensetzung als Futtermittel, als Nahrungsergänzungsmittel oder im medizinischen Bereich und insbesondere in Form von Pulvern, Granulaten, Pastillen, Kapseln, Pellets, Lösungen, Säften oder Geleeprodukten umfasst. Dabei kann es in Abhängigkeit vom jeweiligen konkreten Verwendungsfall durchaus empfehlenswert sein, die Salze und/oder Anlagerungs- und/oder Komplexverbindungen der Guanidinoessigsäure in Kombination mit anderen physiologisch aktiven Wirkstoffen einzusetzen, wobei vor allem Kohlenhydrate, Fette, Aminosäuren, Proteine, Vitamine, Mineralstoffe, Spurenelemente und deren Derivate und beliebige Mischungen daraus besonders geeignet sind.

Gegenstand der vorliegenden Erfindung sind somit auch Futtermittel, Nahrungergänzungsmittel sowie Arzneimittel, welche die erfindungsgemäßen Salze, Anlagerungs- oder Komplexverbindungen enthalten.

Als weiterer Vorteil hat sich in diesem Zusammenhang herausgestellt, dass die Salze und/oder Anlagerungs- und/oder Komplexverbindungen der Guanidinoessigsäure in einem relativ breiten Dosierbereich eingesetzt werden können, wobei sowohl die Einzeldosen als auch die Tagesdosen keinen gravierenden Einschränkungen unterworfen sind. Erfindungsgemäß findet die beanspruchte Verwendung in Einzeldosen von 0,001 bis 1 g/kg Körpergewicht und/oder in Tagesdosen von 0,001 bis 50 g statt.

Falls die erfindungsgemäße Verwendung als Nahrungsergänzungsmittel bei Menschen erfolgt, was vorzugsweise Berücksichtigung findet, kommt insbesondere die Verwendung im Schul-, Sport-, Rekonvaleszenz- und/oder im Geriatrie-Bereich in Frage.

Die Verwendung der erfindungsgemäßen Salze und/oder Anlagerungs- und/oder Komplexverbindungen der Guanidinoessigsäure als Futtermittelzusatz ist insbesondere für Tiere im Leistungssport als bevorzugt anzusehen. Weiterhin können die neuen Salze und/oder Anlagerungs- und/oder Komplexverbindungen der Guanidinoessigsäure als Futterzusatz für Nass- und Trockenfutter für Hunde und Katzen verwendet werden, wobei v. a. positive Auswirkungen auf das Immunsystem und den Allgemeinzustand der Tiere zu vermerken sind.

Weiterhin eignen sich die beanspruchten Salze und/oder Anlagerungs- und/oder Komplexverbindungen der Guanidinoessigsäure auch als Futtermittelzusatz für Zucht- und Masttiere und in diesem Zusammenhang besonders für Schweine, Pferde, Geflügel und Fische, wobei sich die Verwendung als Ersatzmittel für Tier- und/oder Fischmehl sowie daraus hergestellte Produkte als besonders vorteilhaft erwiesen hat. Der Ersatz kann dabei als Teil- oder auch Vollersatz stattfinden.

Entsprechend den bspw. für Kreatin bekannten Verwendungsgebieten können die Salze und/oder Anlagerungs- und/oder Komplexverbindungen der Guanidinoessigsäure im Rahmen der vorliegenden Erfindung auch in kosmetischen oder dermatologischen Zubereitungen verwendet werden. Hierbei ergeben sich aus der hohen Stabilität und Löslichkeit der beanspruchten Verbindungen deutlich Vorteile für die Formulierung, wobei hinsichtlich der Wirksamkeit auch synergistische Effekte zwischen Guanidinoessigsäure und den jeweiligen Reaktionspartnern zu beobachten sind. Als bevorzugt sind Zubereitungen anzusehen, die in Form von Cremes, Lotionen, Sprays, Mousse, wässrigen oder wässrig-ethanolischen Lösungen, Tränkungsmedien für Tücher, wasserfreien oder wasserhaltigen Stiften oder Mikroemulsionen vorliegen. Als ganz besonders bevorzugt ist der topische Anwendungsbereich anzusehen.

Insgesamt bietet die vorliegende Erfindung mit den neuen, stabilen Salzen und/oder Anlagerungs- und/oder Komplexverbindungen der Guanidinoessigsäure weit mehr als nur neue Alternativen zu den bekannten Kreatin-Verbindungen und der freien Guanidinoessigsäure an, da die Eigenschaften der neuen Salze und/oder Anlagerungs- und/oder Komplexverbindungen v. a. in den bevorzugten Anwendungsgebieten die Nachteile der bekannten Verbindungen überwinden und dabei sehr deutliche Verbesserungen darstellen.

Die nachfolgenden Beispiele verdeutlichen die Breite der vorliegenden Erfindung.

### Beispiele

### 1. Nahrungsergänzungsmittel

Nachfolgend sind typische Zusammensetzungen von wohlschmeckenden Formulierungen aufgeführt, deren Bestandteile bei Raumtemperatur einfach trocken abgemischt worden sind. Es wird empfohlen, die pulverförmigen Formulierungen vor deren oraler Aufnahme in 200 ml Fruchtsaft und/oder Wasser zu lösen.

| | | |
|---|---|---|
| 1.1 | 1.500 mg | Glucosamin |
| | 750 mg | Guanidinoessigsäure-α-ketoglutarat |
| | 720 mg | Magnesium-L-hydrogenaspartat |
| | 2.000 mg | Glucose |
| | 500 mg | Ascorbinsäure |
| | | |
| 1.2 | 400 mg | Chondroitinsulfat |
| | 500 mg | Guanidinoessigsäurepyruvat |
| | 2.000 mg | Dicalciumphosphat |
| | 400 mg | (MgCO₃)₄·Mg(OH)₂·5H₂O = ca. 100 Mg |
| | 500 mg | Vitamin C |
| | | |
| 1.3 | 1.000 mg | Glucosamin |
| | 300 mg | Chondroitinsulfat |
| | 2.800 mg | Guanidinoessigsäureaspartat |
| | 3.100 mg | Creatinol-O-phosphat |

### 2. Futtermittelzusatz

2.1. Eine Formulierung bestehend aus 5.000 mg Guanidinoessigsäuremalat und 5.000 mg Inulin wurde in eine typische Rezeptur für Futterpellets zur Futterergänzung von Pferden eingebracht.
2.2 Eine Formulierung bestehend aus 7.000 mg Guanidinoessigsäurelactat, 750 mg Carnitintartrat, 100 mg Succrosestearat, 160 mg Talkum und 1.090 mg Fructose wurde in die Basismasse für Hundebisquits eingebracht.
2.3 Als Masterbatch wurde in eine handelsübliche Katzendosenfuttermischung homogen folgende Formulierung eingebracht: 3.000 mg Guanidinoessigsäurecitrat, 3.000 mg Kreatin, 40 mg Magnesiumstearat, 25 mg Carboxymethylcellulose und 135 mg Lactose.
2.4 Futtermittel für Masthühner Es wurde festgestellt, dass durch den Zusatz von 0,2 Gew.-% Guanidinoessigsäurelipoat (0,2 g/kg) zum luftgetrockneten Futter bei 42 Tagen Mastdauer eine Steigerung des Endgewichts um 5 % gegenüber bisherigen Fütterungsmethoden ohne Guanidinoessigsäure erzielt wurde. Dieser Zuwachs an Gewicht wurde ausschließlich durch Fleischzunahme, jedoch nicht durch Fettzunahme bzw. eine Wassereinlagerung erreicht (Verbesserung des Lean-Body-Mass-Index), wobei das Fleisch auch eine verbesserte Qualität aufwies. Zusätzlich sank der Futtermittelverbrauch um etwa 6 % gegenüber bisherigen Fütterungsmethoden.

### 3. Zubereitungen für kosmetische Cremes

In eine handelsübliche Wasser-in-Öl-Basiscreme werden 1,2 % Guanidinoessigsäurecitrat homogen eingebracht. Die Creme eignet sich u. a. zur Behandlung von sensitiven, defizitären und hypoaktiven Hautzuständen. Weiterhin gegen vorzeitige Hautalterung und umweltbedingte negative Veränderungen der Haut.

## Patentansprüche

1. Verwendung einer Zusammensetzung mit physiologischer Wirksamkeit enthaltend mindestens ein Salz und/oder eine Anlagerungs- und/oder Komplexverbindung der Guanidinoessigsäure mit Äpfelsäure, Ascorbinsäure, Bernsteinsäure, Brenztraubensäure, Fumarsäure, Gluconsäure, α-Ketoglutarsäure, Oxalsäure, Pyroglutaminsäure, 3-Nicotinsäure, Zitronensäure, Maleinsäure, Schwefelsäure, Essigsäure, Ameisensäure, 2-Hydroxybenzoesäure, L-Carnitin, Acetyl-L-Carnitin, Taurin, Betain, Cholin und Liponsäure als wirksamen Inhaltsstoff als Futtermittel oder als Nahrungsergänzungsmittel, insbesondere in Form von Pulvern, Granulaten, Pastillen, Kapseln, Pellets, Lösungen, Säften oder Gelee-Produkten.

2. Verwendung nach Anspruch 1 in Kombination mit anderen physiologisch aktiven Wirkstoffen der Reihe Kohlenhydrate, Fette, Aminosäuren, Proteine, Vitamine, Mineralstoffe, Spurenelemente und deren Derivate und Mischungen daraus.

3. Verwendung nach einem der Ansprüche 1 oder 2 in Einzeldosen von 0,001 bis 1 g/kg Körpergewicht.

4. Verwendung nach einem der Ansprüche 1 bis 3 in Tagesdosen von 0,001 bis 50 g.

5. Verwendung nach einem der Ansprüche 1 bis 4 als Nahrungsergänzungsmittel für Menschen, vorzugsweise im Schul-, Sport-, Rekonvaleszenz- und/oder Geriatrie-Bereich.

## Claims

1. Use of a composition that is physiologically effective containing at least one salt and/or an addition compound and/or a complex compound of guanidinoacetic acid with malic acid, ascorbic acid, succinic acid, pyruvic acid, fumaric acid, gluconic acid, α-ketoglutaric acid, oxalic acid, pyroglutamic acid, 3-nicotinic acid, citric acid, maleic acid, sulfuric acid, acetic acid, formic acid, 2-hydroxybenzoic acid, L-carnitine, acetyl-L-carnitine, taurine, betaine, choline and lipoic acid as the active ingredient as an animal feed or as a dietary supplement especially in the form of powders, granulates, lozenges, capsules, pellets, solutions, juices or jelly products.

2. Use according to claim 1 in combination with other physiologically active substances from the group comprising carbohydrates, fats, amino acids, proteins, vitamins, mineral substances, trace elements and derivatives thereof and mixtures thereof.

3. Use according to one of the claims 1 or 2 in single doses of 0.001 to 1 g/kg body weight.

4. Use according to one of the claims 1 to 3 in daily doses of 0.001 to 50 g.

5. Use according to one of the claims 1 to 4 as a dietary supplement for humans preferably in the school, sport, convalescence and/or in the geriatric field.

## Revendications

1. Utilisation d'une composition ayant une activité physiologique, contenant au moins un sel et/ou un composé d'addition et/ou un complexe de l'acide guanidinoacétique avec l'acide malique, l'acide ascorbique, l'acide succinique, l'acide pyruvique, l'acide fumarique, l'acide gluconique, l'acide α-cétoglutarique, l'acide oxalique, l'acide pyroglutaminique, l'acide 3-nicotinique, l'acide citrique, l'acide maléique, l'acide sulfurique, l'acide acétique, l'acide formique, l'acide 2-hydroxybenzoïque, la L-carnitine, l'acétyl-L-carnitine, la taurine, la bétaïne, la choline et l'acide lipoïque en tant que constituant actif, en tant que produit alimentaire ou en tant que complément alimentaire, en particulier sous la forme de poudres, de granulés, de pastilles, de capsules, de pellets, de solutions, de jus ou de produits en gelée.

2. Utilisation selon la revendication 1, en combinaison avec d'autres agents actifs du point de vue physiologique de la série des glucides, des graisses, des aminoacides, des protéines, des vitamines, des substances minérales, des oligoéléments et de leurs dérivés et de leurs mélanges.

3. Utilisation selon l'une quelconque des revendications 1 ou 2, en doses individuelles de 0,001 à 1 g/kg poids corporel.

4. Utilisation selon l'une quelconque des revendications 1 à 3 en doses journalières de 0,001 à 50 g.

5. Utilisation selon l'une quelconque des revendications 1 à 4, en tant que complément alimentaire pour l'Homme, de préférence dans le domaine scolaire, sportif, de la convalescence et/ou de la gériatrie.
